# EUROPEAN PATENT SPECIFICATION

(11) **EP 2 310 379 B1**
(45) Date of publication and mention of the grant of the patent: **16.12.2015**
(21) Application number: 09772614.5
(22) Date of filing: 18.06.2009
(51) Int. Cl.: C07D 311/26

(54) **METHOD FOR THE FRACTIONATION OF KNOTWOOD EXTRACT AND USE OF A LIQUID-LIQUID EXTRACTION FOR PURIFICATION OF KNOTWOOD EXTRACT**
VERFAHREN ZUR FRAKTIONIERUNG VON KNOTENHOLZEXTRAKT UND ANWENDUNG EINER FLÜSSIG-FLÜSSIGEXTRAKTION ZUR AUFREINIGUNG VON KNOTENHOLZEXTRAKT
PROCÉDÉ POUR LE FRACTIONNEMENT D'EXTRAIT DE BOIS NOUEUX ET UTILISATION D'UNE EXTRACTION LIQUIDE-LIQUIDE POUR LA PURIFICATION D'EXTRAIT DE BOIS NOUEUX

(30) Priority: 01.07.2008 FI 20085681
(43) Date of publication of application: 20.04.2011
(73) Proprietor: UPM-Kymmene Oyj, 00100 Helsinki (FI)
(72) Inventor: PIETARINEN, Suvi, FI-53100 Lappeenranta (FI); HOTANEN, Ulf, FI-53810 Lappeenranta (FI)
(74) Representative: Boco IP Oy Ab
(86) International application number: PCT/FI2009/050544
(87) International publication number: WO 2010/000927

(56) References cited:
- WO-A-02/098830
- WO-A-2005/047423
- FRANCOIS SIMARD, JEAN LEGAULT, SERGE LAVOIE, VAKTHANG MSHVILDADZE, ANDRE PICHETTE: "Isolation and identification of cytotoxic compounds from the wood of Pinus resinosa" PHYTOTHERAPY RESEARCH, vol. 22, 2008, pages 919-922, XP002548831
- SUVI P PIETARINEN ET AL: "Knotwood and bark extracts: strong antioxidants from waste materials" JOURNAL OF WOOD SCIENCE ; OFFICIAL JOURNAL OF THE JAPAN WOOD RESEARCH SOCIETY, SPRINGER-VERLAG, TO, vol. 52, no. 5, 15 March 2006 (2006-03-15), pages 436-444, XP019431856 ISSN: 1611-4663

## Description

### FIELD OF THE INVENTION

The present invention relates to a method for the fractionation of knotwood extract, which has been obtained by extraction of knotwood with a hydrophilic solvent. The invention also relates to the use of a liquid-liquid extraction for the purification of hydrophilic knotwood extract. The present process provides a purified knotwood extract, which contains more than 90 % lignans, flavonoids and stilbenes and less than 10 % impurities selected from resin acids, fatty acids, sterols, juvabiones, triglycerides and combinations thereof, especially abietic acid derivatives (neoabietine, abietine, dehydroabietine) and pimaric acid.

### BACKGROUND OF THE INVENTION

Knotwood, i.e. the inner branch stubs seated within the tree, contains an abundance of extractive substances, the utility of which has been studied intensively. Among these compounds, especially lignans of coniferous trees such as spruce, pine and fir have become known as hormonal anticancer compounds.

Heartwood as well as knotwood of coniferous trees contain large amounts of pitch, which consists primarily of resin and rosin acids and terpenes. The knotwood and heartwood additionally contains phenolic compounds, the most abundant of which are the above mentioned lignans as well as pinosylvin and its mono methyl ether.

The phenolic extracts of knotwood are known to be strong antioxdants and they may be used as substitutes for synthetic antioxidants, for instance, in food products, cosmetics, pharmaceuticals, etc.

In the prior art, the desired components of the heartwood and especially of the knotwood has been typically extracted with a hydrophilic solvent such as ethanol.

WO 02/098830 (Holmbom et al.) discloses a method for isolating phenolic substances or juvabiones from knotwood by extracting knotwood with a polar solvent having a dielectric constant greater than 3 at 25 °C. The solvents mentioned in the document are hydrophilic solvents such as water or a mixture of water and an alcohol or acetone. The preferred hydrophilic solvent is water and ethanol. The document also mentions sequential extraction so that the lipophilic extractives are first extracted from the knotwood with hexane and then the remaining hydrophilic extractives are extracted from the wood with acetone/water. A report on the hydrophilic extractives is provided in the document.

WO 2005/047423 (Arbonova) relates to the use of a hydrophilic extract of knotwood as an antioxidative agent. The polar solvent comprises water or a mixture of water and alcohol or acetone. A sequential extraction with a lipophilic and then a hydrophilic solvent may be used for selective extraction of the hydrophilic components. The hydrophilic extract may be purified by chromatography or crystallization. According to the document, it is also possible to remove the lipophilic extractives from an acetone extract by refluxing the dry acetone extract with hexane.

The prior art process of extracting the desired hydrophilic compounds from knotwood is not altogether satisfactory. The hydrophilic extraction is typically performed with ethanol/water and the resulting extract contains also lipophilic pitch components besides the desired hydrophilic components. The lipophilic impurities impair the quality of the hydrophilic extract. Sometimes the impurities may even prohibit the use of the extract for an intended purpose. It has, for instance, been noted that the resin acids of pine are strong allergens and their presence in a pine extract prohibits the use of the extract in the cosmetics industry. The lipophilic juvabiones contained in hydrophilic fir extract are phytoestrogens and their presence therefore limits the use of the extract. On the other hand, juvabiones may be used in insect control preparations.

The hydrophilic extract can be purified by technical means such as chromatography or crystallization and the amount of lipophilic extractives can be reduced by a sequential lipophilic/hydrophilic extraction of the knotwood material. However, there is still a need for a simple and cost effective way of increasing the purity of the hydrophilic knotwood extract. There is also a need for providing a lipophilic extract for use in industrial applications. The present invention sets out to satisfy these needs and to provide a new process for purifying knotwood extract.

### SUMMARY OF THE INVENTION

The present invention relates to a method for the fractionation of knotwood extract, wherein knotwood is extracted with a hydrophilic solvent, the resulting knotwood extract is extracted with a lipophilic solvent, and at least one knotwood extract fraction is recovered.

The first extraction provides a crude hydrophilic extract. The second extraction or fractionation provides a purified hydrophilic extract and a lipophilic extract. The fractionation is preferably performed as a liquid-liquid extraction of the crude hydrophilic extract with a lipophilic solvent.

The lipophilic solvent typically comprises a non-polar solvent or a mixture of nonpolar solvents having a dielectric constant below 3, determined at 25 °C, while the hydrophilic solvent comprises a polar solvent or a mixture of polar solvents having a dielectric constant greater than 3, and preferably greater than 15, determined at 25 °C.

The knotwood used in the method according to the invention may be derived from different wood species containing hydrophilic and lipophilic extractives. However, the knotwood typically comprises knots of coniferous wood, preferably wood selected from pine (*Pinus*), spruce (*Picea*), fir (*Abies*) and combinations thereof.

The present invention also relates to the use of a lipophilic liquid-liquid extraction for the purification of hydrophilic knotwood extract. The purification according to the invention removes lipophilic impurities from a hydrophilic knotwood extract. The degree to which the lipophilic impurities are removed depends on the fractionation conditions and sequences. The fractionation typically removes resin components such as abietic acid and its derivatives, and pimaric acid.

In one embodiment of the invention, the fractionation provides a hydrophilic knotwood extract having less than 25%, preferably less than 10% and most preferably less than 5% impurities selected from the group consisting of resin acids, fatty acids, sterols, juvabiones, triglycerides and combinations thereof. In a preferred embodiment, said extract contains less than 10 % and preferably less than 5 % of resin acids, which are selected from the group consisting of neoabietic acid, abietic acid" dehydroabietic acid, pimaric acid, isopimaric acid, palustric acid and combinations thereof.

The present invention typically provides a purified hydrophilic knotwood extract, which contains more than 90 %, preferably more than 95% and most preferably more than 99% of components selected from lignan(s), flavonoid(s), stilbene(s) and combinations thereof, and less than 10 %, preferably less than 5 % and most preferably less than 1 % of impurities selected from resin acids, fatty acids, sterols, juvabiones, triglycerides and combinations thereof.

### BRIEF DESCRIPTION OF THE DRAWINGS

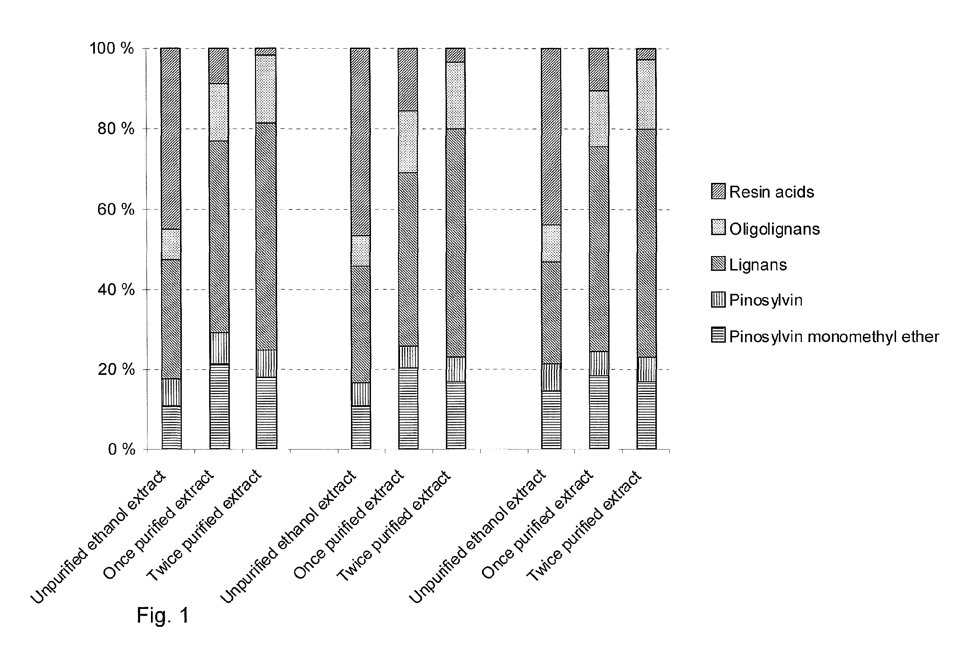

Fig. 1 shows the influence of repeated lipophilic liquid-liquid extractions on the resin acid content of a hydrophilic knotwood extract.

### DETAILED DESCRIPTION OF THE INVENTION

The present invention is based on the realization that the quality of the traditional hydrophilic knotwood extract may be improved by performing a lipophilic liquid-liquid fractionation of the hydrophilic knotwood extract. The purification of the knotwood extract facilitates its commercialization and makes available a new and improved alternatives for the fractionation and purification of extracts from various wood species. The invention also enables the production of a lipophilic knotwood extract from which resin acids and other pitch components may be recovered.

In the method according to the invention, knotwood is extracted with a hydrophilic solvent to provide a crude hydrophilic knotwood extract. The crude hydrophilic extract is extracted with a lipophilic solvent to provide a purified hydrophilic extract and a lipophilic extract. One or both of the resulting extracts may be recovered. The extracts may be used as such or may be treated further by isolation of single components or groups of components.

The crude hydrophilic extract may be concentrated, purified or fractionated before the lipophilic extraction by removal of one or more components. If two hydrophilic fractions are provided, the liquid-liquid extraction of the present invention can be applied to any one or both of the thus obtained fractions to remove lipophilic components there from.

In the prior art a combination of a hydrophilic and a lipophilic solvent in the extraction of knotwood has been suggested. The extraction has been performed as a sequential extraction by first extracting the knotwood with hexane for removing lipophilic extractives from the wood material. There after the once extracted wood material has been subjected to a second extraction with a hydrophilic solvent such as a mixture of acetone and water. However, this sequential extraction has certain drawbacks which are obviated by the present invention.

Thus, for extraction with a lipophilic solvent, the wood material must be dry so that the lipophilic solvent can penetrate into the wood. Drying the wood material is time and energy consuming. When the wood material is extracted with a hydrophilic solvent such as water, ethanol or acetone, the wood material need not be dried before the extraction. On the contrary, water is typically a component of the solvent itself. As a consequence, the wood extracting plant can be smaller and easier to run and requires less energy than if the wood is extracted with a lipophilic solvent.

When the initial extraction of the wood material is performed with a hydrophilic solvent, the hydrophilic extract may be concentrated prior to the lipophilic liquid-liquid extraction to reduce the volume of the hydrophilic extract. This reduces the volume of lipophilic solvent required compared to a situation, wherein the lipophilic extraction is performed on the wood material. Reducing the amount of solvent also improves the safety of the process.

When the wood material is initially extracted with a lipophilic solvent, as in the prior art, part of the desired hydrophilic components will also be transferred into the extracting lipophilic solvent. This reduces the yield of hydrophilic extract obtainable in the subsequent hydrophilic extraction sequence.

When the wood is extracted with a hydrophilic solvent, a large portion of the resin and fatty acids remains unextracted in the wood material. Since these components are typically not desired, it is advantageous to leave them in the wood. The hydrophilic extract will contain only a minor portion of the resin and fatty acids of the wood and, consequently, the contamination of the lipophilic solvent with resin and fatty acids is smaller, which facilitates the purification and recycling of the lipophilic solvent and reduces the size of the recovery process.

The prior art also has suggested extracting stilbenes from knotwood with acetone and removing the lipophilic extractives by refluxing the dried acetone extract with hexane. However, this method requires first drying of the obtained acetone extract and then leaching the solid extract with hexane. In order to obtain sufficient extraction by leaching there has to be full contact between the solid acetone extract and the liquid hexane. Although such a process may work in a laboratory, it is not suitable for operation on an industrial scale.

The hydrophilic solvent used in the prior art for extracting knotwood has been defined as a polar solvent or a mixture of polar solvents having a dielectric constant greater than 3. However, no solvents with a dielectric constant below 15 have been shown to operate as polar solvents in the desired manner. The solvents typically used in the prior art include water, alcohol, acetone and mixtures thereof. The dielectric constant of water is about 80, that of ethanol is about 25 and that of acetone is about 20.

The solvent used according to the present invention for the liquid-liquid extraction is a non-polar solvent, which is preferably not miscible with the polar solvent or only to a very minor degree miscible with the polar solvent used in the hydrophilic extraction.

The preferred non-polar solvent or mixture of non-polar solvents has a dielectric constant below 3, determined at 25 °C. Such solvents include but are not limited to hexane, heptane, benzene, toluene, cyclohexane, cyclopentane and mixtures thereof. The approximative dielectric constant of hexane is 2.0, heptane 1.9, benzene 2.3, toluene 2.4, cyclohexane 2.0 and cyclopentane 2.0.

The skilled person is able to select other suitable hydrophilic and lipophilic solvents based on his knowledge of their polarity and other properties.

In the method according to the invention, the extraction with a hydrophilic solvent is typically performed on non-dried or only slightly dried knotwood, since water in itself is a polar solvent for the desired hydrophilic extractives. Preferably the wood material has a dry solids content of 60 to 95 %, preferably 70 to 90 %.

The liquid-liquid extraction according to the present invention is facilitated by the presence of water in the hydrophilic extract. The hydrophilic solvent used for the extraction of the knotwood typically contains water. To facilitate the phase separation of said lipophilic solvent extraction, the amount of water in said hydrophilic solvent and/or in the resulting crude hydrophilic extract may be adjusted. The water may also be added with the lipophilic solvent.

In an embodiment of the invention, the hydrophilic solvent comprises ethanol and the lipophilic solvent comprises heptane, and the water content of the hydrophilic extract subjected to the liquid-liquid extraction is 10 % or more. Preferably, the water content is between 10 and 30 % on a volume/volume basis. Water in admixture with the ethanol lowers the solubility of the lipophilic extractives in the ethanol. However, the total water content of ethanol during extraction of the wood should not rise above 50%, because this reduces the yield of the desired hydrophilic extractives in the solvent.

In order to improve the purification of the hydrophilic extract and to remove more of the lipophilic extractives, the extraction with a lipophilic solvent is repeated at least once. Preferably the liquid-liquid extraction is repeated twice.

The once extracted (purified) hydrophilic extract may be treated by other means between consecutive lipophilic extractions. Thus, for instance, the water content of the extract may be adjusted, the volume of the extract may be reduced or increased and components may be removed by crystallization or other means between extraction stages. An intermediate and/or final extraction may also be performed with a lipophilic solvent different from the lipophilic solvent used in the first lipophilic liquid-liquid extraction. Although adjusting the water content and/or changing the solvent may complicate the extraction process, advantages may be obtained in the product profile and purity, which may in certain cases justify such extra steps.

In the typical operation of the process according to the invention, the hydrophilic extract is subjected to three consecutive liquid-liquid extractions with the same lipophilic solvent being used in all the extraction steps. After each extraction, the lipophilic solvent is recovered and recirculated to a subsequent lipophilic extraction. The recovery of the solvent is typically done by evaporation. Certain impurities, such as terpenes are difficult to remove from the lipophilic solvent and the solvent needs to be purified or fresh make-up solvent needs to be added to the solvent circulation from time to time to prevent accumulation of high amounts of impurities.

The hydrophilic solvent used in the hydrophilic solvent extraction may also be recovered from the purified hydrophilic extract. The hydrophilic solvent is preferably recovered by evaporation and recycled to a subsequent hydrophilic extraction step.

The method according to the invention allows recovery of both lipophilic extract fractions and hydrophilic extract fractions. When the recovered knotwood extract fraction comprises one or more lipophilic fractions, the lipophilic solvent is typically removed by evaporation, and the resulting lipophilic resin is recovered. The lipophilic fractions are typically combined before evaporation.

When the recovered knotwood extract fraction comprises one or more hydrophilic fractions, the hydrophilic solvent is typically removed by evaporation and a purified hydrophilic extract is recovered. The purified fraction or the combined fractions may be treated further in a manner known *per se* to release specific component(s) thereof. The purified fraction may also be further purified by chromatography, crystallization, or the like.

Although most trees and many other plants contain lignans, the coniferous wood species are known to be exceptionally rich in lignans having desired properties.

Thus, the preferred knotwood used in the present invention comprises knots of coniferous wood, and most preferably knotwood selected from pine (*Pinus*)*,* spruce (*Picea*)*,* fir (*Abies*) and combinations thereof.

The crude hydrophilic extract obtained by hydrophilic extraction of such coniferous knotwood contains components selected from the group consisting of lignan(s), flavonoid(s), stilbene(s), juvabione(s), fatty acid(s), resin acid(s), sterol(s), triglyceride(s) and combinations thereof.

The extraction with a lipophilic solvent removes resin acids, fatty acids, sterols juvabiones and triglycerides so that the purified hydrophilic extract obtained by the lipophilic extraction according to the invention contains mainly components selected from the group consisting of lignan(s), flavonoid(s), stilbene(s) and combinations thereof. The amount of said components is typically more than 90% of the dry solids content.

The lignans are typically selected from allo-hydroxymatairesino, (-)- hydroxymatairesinol, secoisolariciresinol, conidendrin, conidendric acid, todolactol, isoliovil, lariciresinol, lignan A, matairesinol, oligolignans, nortrachelogenin and pinoresinol. The stilbenes are typically selected from pinosylvin and pinosylvin monomethyl ether.

The extraction with a lipophilic solvent according to the invention provides a lipophilic extract, which includes components selected from resin acids, fatty acids, sterols, juvabiones and triglycerides. The most abundant resin components of coniferous knotwood extract are selected from the group consisting of neoabietine, abietine, dehydroabietine, palustric acid, pimaric acid and isopimaric acid.

The present invention provides a novel use of a lipophilic liquid-liquid extraction for the purification of hydrophilic knotwood extract. By repetition of the liquid-liquid extraction sequence, very low amounts of impurities are obtainable. According to an embodiment or the invention, the use of the liquid-liquid extraction provides a purified hydrophilic knotwood extract having less than 25%, preferably less than 10% and most preferably less than 5% impurities selected from the group consisting of resin acids, fatty acids, sterols, juvabiones, triglycerides and combinations thereof. Said impurities are typically selected from the group consisting of neoabietic acid, abietic acid, dehydroabietic acid, pimaric acid, isopimaric acid, palustric acid and combinations thereof.

According to an embodiment of the invention, the purifying fractionation provides a purified hydrophilic extract product, which contains more than 90 %, preferably more than 95% and most preferably more than 99% of components selected from lignan(s), flavonoid(s), stilbene(s) and combinations thereof and less than 10 %, preferably less than 5 % and most preferably less than 1 % of impurities selected from resin acids, fatty acids, sterols, juvabiones, triglycerides and combinations thereof.

The purified extract typically consists essentially of 55-75 % of lignans, 20-40% of stilbenes, and 1 to 5% of said impurities.

The lignans are selected from allo-hydroxymatairesino, (-)-hydroxymatairesinol, secoisolariciresinol, conidendrin, conidendric acid, todolactol, isoliovil, lariciresinol, lignan A, matairesinol, oligolignans, nortrachelogenin and pinoresinol, and the stilbenes are selected from pinosylvin and pinosylvin monomethyl ether. One or more of said compounds may be isolated from said extract.

Among the impurities, fatty acids with 18 carbon atoms (C18:0 and C18:2), pimaric acid, isopimaric acid, palustric acid, dehydroabietic acid, abietic acid and neoabietic acid are the most abundant.

In one embodiment of the invention, the purified knotwood extract consists essentially of 20-40% of the dry solids of stilbenes selected from pinosylvin and pinosylvin monomethyl ether, 55-75 % of the dry solids of lignans selected from allo-hydroxymatairesino, (-)-hydroxymatairesinol, secoisolariciresinol, conidendrin, conidendric acid, todolactol, isoliovil, lariciresinol, lignan A, matairesinol, oligolignans, nortrachelogenin and pinoresinol, and 1 to 5% of the dry solids of impurities selected from C18 fatty acids, neoabietic aicd, abietic acid, dehydroabietic acid, pimaric acid, isopimaric acid and palustric acid.

In the present specification and claims, the percentages of the components in the extracts are calculated on the dry solids content unless otherwise specified and the following terms have the meanings defined below.

The term "knotwood" refers to wood material which includes knots, i.e. branches and especially the inner extensions of branches into the stem of a tree. The knotwood is harder than the rest of the wood and it is typically obtained by selection of over-sized chips in the production of chips for pulp and paper processes.

The term "liquid-liquid extraction" refers to an extraction process which uses two solvents, which are both in the liquid phase, i.e. at a temperature below their boiling points. The two solvents are immiscible with each other in the sense that they can be separated after the extraction process. Liquid-liquid extraction is also known as "solvents extraction". The aim is to separate compounds based on their relative solubilities in the two different solvents. The process extracts one or more substances from one liquid phase into another liquid phase.

The term "knotwood extract" refers to an extract of knotwood obtained when knotwood has been extracted with a solvent. The extract is initially in a liquid form in the extracting solvent medium and the solid extract is typically obtained by evaporation of the solvent.

The term "knotwood extract fraction" refers to a fraction obtained after a liquid-liquid extraction has been performed on a knotwood extract to fractionate the same. The knotwood extract fraction in question may be hydrophilic or lipophilic.

The term "crude hydrophilic extract" refers to an extract obtained when knotwood is initially extracted with a hydrophilic solvent. Hydrophilic extraction of the knotwood may be performed several times, in which case the obtained extracts are typically combined.

The term "purified hydrophilic extract" refers to an extract fraction which has been obtained by subjecting the crude hydrophilic extract or a purified form thereof to liquid-liquid extraction in accordance with the present invention.

The term "lipophilic extract" refers to an extract fraction which has been obtained by subjecting a crude or purified hydrophilic extract to liquid-liquid extraction so that lipophilic components of said hydrophilic extract have been transferred into the lipophilic solvent.

The following example is provided to further illustrate the invention and is not intended to limit the scope thereof. Based on the above description, a person skilled in the art will be able to modify the invention in many ways to provide purified hydrophilic and/or lipophilic extracts and isolated components thereof with a wide range of defined properties.

### Example 1

Ground knotwood of pine and spruce was mixed and three different mixtures were extracted with boiling ethanol containing 5 % water. The water content of the knotwood mixtures was about 50 % of the total weight. The amount of ethanol used was about four times that of the knots. The hydrophilic extraction was continued for about 3 hours at 80 °C.

The resulting crude hydrophilic extracts were analysed and were found to contain approximately 55 % of the dry solids of the desired hydrophilic extractives, namely lignans and stilbenes.

The crude hydrophilic extracts in their respective ethanol/water solvents were each subjected to three consecutive liquid-liquid extractions with heptane in a reactor equipment at a temperature of 50 °C. In each extraction, the liquid was first stirred and then left to separate for one hour. The hydrophilic solvent phase was analyzed to show the amount of impurities remaining in the purified extract.

The results obtained by gas chromatographic analysis are indicated graphically in Fig. 1 and numerically in Table 1 .

**Table 1: Concentrations of the identified compounds**

| | **Unpurified ethanol extract** | **Once purified extract** | **Twice purified extract** | **Unpurified ethanol extract** | **Once purified extract** | **Twice purified extract** | **Unpurified ethanol extract** | **Once purified extract** | **Twice purified extract** |
|---|---|---|---|---|---|---|---|---|---|
| **Pinosylvin monomethyl ether** | **11 %** | **21 %** | **18 %** | **11 %** | **21 %** | **17 %** | **15 %** | **18 %** | **17 %** |
| **Pinosylvin** | 7 % | 8 % | 7 % | 6 % | 5 % | 6 % | 7 % | 6 % | 6 % |
| acid 18:0 ja 18:2 | 4 % | 1 % | 1 % | 6 % | 2 % | 1 % | 7 % | 2 % | 1 % |
| pimaric acid | 4 % | 2 % | 0 % | 5 % | 3 % | 1 % | 4 % | 2 % | 0 % |
| isopimaric acid | 3 % | 0 % | 0 % | 0 % | 0 % | 0 % | 3 % | 0 % | 0 % |
| palustric acid | 7 % | 0 % | 0 % | 6 % | 0 % | 0 % | 0 % | 0 % | 0 % |
| dehydroabietic acid | 7 % | 2 % | 1 % | 6 % | 6 % | 1 % | 9 % | 4 % | 1 % |
| abietic acid | 12 % | 3 % | 0 % | 17 % | 5 % | 1 % | 15 % | 3 % | 0 % |
| Neoabietic acid | 7 % | 1 % | 0 % | 6 % | 0 % | 0 % | 6 % | 0 % | 0 % |
| **Resin acids** | **45 %** | **9 %** | **2 %** | **46 %** | **16 %** | **4 %** | **44 %** | **11 %** | **3 %** |
| **Lignans** | **30 %** | **48 %** | **57 %** | **29 %** | **43 %** | **57 %** | **26 %** | **51 %** | **57 %** |
| dimers | 5 % | 8 % | 12 % | 5 % | 14 % | 12 % | 4 % | 8 % | 13 % |
| trimers | 1 % | 4 % | 3 % | 2 % | 1 % | 3 % | 3 % | 3 % | 3 % |
| tetramers | 1 % | 2 % | 2 % | 1 % | 0 % | 1 % | 2 % | 3 % | 1 % |
| **Oligolignans** | **7 %** | **14 %** | **17 %** | **8 %** | **16 %** | **17 %** | **9 %** | **14 %** | **17 %** |

As can be seen from Fig. 1 and Table 1, the liquid-liquid extraction according to the invention provides an excellent tool for purifying a hydrophilic knotwood extract from lipophilic impurities

The present invention has been described herein with reference to specific embodiments. It is however clear to those skilled in the art that the process(es) may be varied within the bounds of the claims.

## Claims

1. A method for the fractionation of knotwood extract, **characterized in that** knotwood is initially extracted with a hydrophilic solvent, the resulting knotwood extract is extracted with a lipophilic solvent, at least one knotwood extract fraction is recovered and said fractionation is performed as a liquid-liquid extraction.

2. The method according to claim 1, wherein said lipophilic solvent comprises a non-polar solvent or a mixture of non-polar solvents having a dielectric constant below 3, determined at 25 °C.

3. The method according to claim 2, wherein said lipophilic solvent is selected from the group consisting of hexane, heptane, benzene, toluene, cyclohexane, cyclopentane and mixtures thereof.

4. The method according to claim 1, wherein said hydrophilic solvent comprises a polar solvent or a mixture of polar solvents having a dielectric constant greater than 3, and preferably greater than 15, determined at 25 °C.

5. The method according to claim 4, wherein said hydrophilic solvent is selected from the group consisting of water, alcohol, acetone and mixtures thereof.

6. The method according to claim 1, wherein said extraction with a hydrophilic solvent is performed on non-dried or partially dried knotwood material having a dry solids content of 60 to 95 %, preferably 70 to 90 %.

7. The method according to any one of the preceding claims, wherein the hydrophilic solvent used for said extraction contains water.

8. The method according to claim 7, wherein the amount of water in said hydrophilic solvent and/or in the resulting hydrophilic knotwood extract is adjusted to facilitate the phase separation of said lipophilic solvent extraction, preferably said hydrophilic solvent comprises ethanol, said lipophilic solvent comprises heptane and said water content is 10 % or more.

9. The method according to any one of the preceding claims, wherein said extraction with a lipophilic solvent is repeated at least once.

10. The method according to any one of the preceding claims, wherein said recovered knotwood extract fraction comprises one or more lipophilic fractions and said lipophilic solvent is removed by evaporation, and wherein the resulting lipophilic resin is recovered.

11. The method according to any one of the preceding claims, wherein said recovered knotwood extract fraction comprises one or more hydrophilic fractions and said hydrophilic solvent is removed by evaporation, and wherein a purified hydrophilic extract is recovered and is optionally treated further to release specific component(s) thereof.

12. The method according to any one of the preceding claims, wherein said knotwood comprises knots of coniferous wood, preferably wood selected from pine (*Pinus*), spruce (*Picea*), fir *(Abies)* and combinations thereof.

13. The method according to claim 12, wherein said extraction with a hydrophilic solvent provides a crude hydrophilic extract containing component(s) selected from the group consisting of lignan(s), flavonoid(s), stilbene(s), juvabione(s), fatty acid(s), resin acid(s), sterol(s), triglyceride(s) and combinations thereof.

14. The method according to claim 12, wherein said extraction with a lipophilic solvent provides a purified hydrophilic extract containing more than 90 % of the dry solids of component(s) selected from the group consisting of lignan(s), flavonoid(s), stilbene(s) and combinations thereof.

15. The method according to claim 14, wherein said lignans are selected from allo-hydroxymatairesino, (-)-hydroxymatairesinol, secoisolariciresinol, conidendrin, conidendric acid, todolactol, isoliovil, lariciresinol, lignan A, matairesinol, oligolignans, nortrachelogenin, pinoresinol and combinations thereof, and said stilbenes are selected from pinosylvin, pinosylvin monomethyl ether and combinations thereof, and wherein one or more of said compounds is/are isolated from said extract.

16. The method according to any one of the preceding claims, wherein said lipophilic solvent is recovered after said extraction and recirculated to a subsequent lipophilic extraction.

17. The method according to any one of the preceding claims, wherein said extraction with a lipophilic solvent provides a lipophilic extract, which includes components selected from the group consisting of resin acid(s), fatty acid(s), sterol(s), juvabione(s), triglyceride(s) and combinations thereof.

18. Use of a lipophilic liquid-liquid extraction for the purification of hydrophilic knotwood extract, wherein said hydrophilic knotwood extract is obtained by an initial extraction of knotwood with a hydrophilic solvent.

19. The use of claim 18, wherein said purification provides a hydrophilic knotwood extract having less than 25%, preferably less than 10% and most preferably less than 5% of the dry solids of impurities selected from the group consisting of resin acids, fatty acids, sterols, juvabiones, triglycerides and combinations thereof.

20. A purified knotwood extract obtainable by liquid-liquid extraction, **characterized in that** it contains more than 90 %, preferably more than 95% and most preferably more than 99% of the dry solids of components selected from combinations of lignan(s), flavonoid(s) and/or stilbene(s), and less than 10 %, preferably less than 5 % and most preferably less than 1 % of the dry solids of impurities selected from resin acids, fatty acids, sterols, juvabiones, triglycerides and combinations thereof.

21. The purified knotwood extract according to claim 20, which consists essentially of 20-40% of the dry solids of stilbenes selected from pinosylvin and pinosylvin monomethyl ether, 55-75 % of the dry solids of lignans selected from allo-hydroxymatairesino, (-)-hydroxymatairesinol, secoisolariciresinol, conidendrin, conidendric acid, todolactol, isoliovil, lariciresinol, lignan A, matairesinol, oligolignans, nortrachelogenin and pinoresinol, and 1 to 5% of the dry solids of impurities selected from C18 fatty acids, neoabietic aicd, abietic acid, dehydroabietic acid, pimaric acid, isopimaric acid and palustric acid.

## Patentansprüche

1. Verfahren zur Fraktionierung von Knotenholzextrakt, **dadurch gekennzeichnet, dass** Knotenholz anfänglich mit einem hydrophilen Lösungsmittel extrahiert wird, das resultierende Knotenholzextrakt mit einem lipophilen Lösungsmittel extrahiert wird, wenigstens eine Knotenholzextrakt-Fraktion zurückgewonnen wird und die Fraktionierung als Flüssig-Flüssig-Extraktion durchgeführt wird.

2. Verfahren nach Anspruch 1, wobei das lipophile Lösungsmittel ein nichtpolares Lösungsmittel oder ein Gemisch aus nichtpolaren Lösungsmitteln umfasst, das eine dielektrische Konstante von unter 3 aufweist, die bei 25 °C bestimmt wird.

3. Verfahren nach Anspruch 2, wobei das lipophile Lösungsmittel aus der Gruppe gewählt ist, die aus Hexan, Heptan, Benzol, Toluol, Cyclohexan, Cyclopentan und deren Gemischen besteht.

4. Verfahren nach Anspruch 1, wobei das hydrophile Lösungsmittel ein polares Lösungsmittel oder ein Gemisch aus polaren Lösungsmitteln umfasst, das eine dielektrische Konstante von über 3, und vorzugsweise über 15, aufweist, die bei 25 °C bestimmt wird.

5. Verfahren nach Anspruch 4, wobei das hydrophile Lösungsmittel aus der Gruppe gewählt ist, die aus Wasser, Alkohol, Aceton und deren Gemischen besteht.

6. Verfahren nach Anspruch 1, wobei die Extraktion mit einem hydrophilen Lösungsmittel an ungetrocknetem oder teilgetrocknetem Knotenholzmaterial durchgeführt wird, das einen Trockensubstanzgehalt von 60 bis 95 %, vorzugsweise 70 bis 90 %, aufweist.

7. Verfahren nach einem der vorhergehenden Ansprüche, wobei das für die Extraktion verwendete hydrophile Lösungsmittel Wasser enthält.

8. Verfahren nach Anspruch 7, wobei die Wassermenge im hydrophilen Lösungsmittel und/oder im resultierenden hydrophilen Knotenholzextrakt so eingestellt wird, dass sie die Phasentrennung der lipophilen Lösungsmittelextraktion begünstigt, wobei vorzugsweise das hydrophile Lösungsmittel Ethanol umfasst, das lipophile Lösungsmittel Heptan umfasst und der Wassergehalt 10 % oder mehr beträgt.

9. Verfahren nach einem der vorhergehenden Ansprüche, wobei die Extraktion mit einem lipophilen Lösungsmittel wenigstens ein Mal wiederholt wird.

10. Verfahren nach einem der vorhergehenden Ansprüche, wobei die rückgewonnene Knotenholzextrakt-Fraktion eine oder mehrere lipophile Fraktionen umfasst und das lipophile Lösungsmittel durch Verdampfung entfernt wird, und wobei das resultierende lipophile Harz rückgewonnen wird.

11. Verfahren nach einem der vorhergehenden Ansprüche, wobei die rückgewonnene Knotenholzextrakt-Fraktion eine oder mehrere hydrophile Fraktionen umfasst und das hydrophile Lösungsmittel durch Verdampfung entfernt wird, und wobei ein gereinigter hydrophiler Extrakt rückgewonnen wird und optional weiterbehandelt wird, um einen oder mehrere spezielle Bestandteile herauszulösen.

12. Verfahren nach einem der vorhergehenden Ansprüche, wobei das Knotenholz Knoten von Nadelholz umfasst, vorzugsweise Holz gewählt aus Kiefer (*Pinus*), Fichte (*Picea*), Tanne (*Abies*) und deren Kombinationen.

13. Verfahren nach Anspruch 12, wobei die Extraktion mit einem hydrophilen Lösungsmittel einen hydrophilen Rohextrakt bereitstellt, der Bestandteil(e) enthält, die gewählt sind aus der Gruppe bestehend aus Lignan(en), Flavonoid(en), Stilben(en), Juvabion(en), Fettsäure(n), Harzsäure(n), Sterol(en), Triglycerid(en) und deren Kombinationen.

14. Verfahren nach Anspruch 12, wobei die Extraktion mit einem lipophilen Lösungsmittel einen gereinigten hydrophilen Extrakt bereitstellt, der mehr als 90 % Trockensubstanz an Bestandteil(en) enthält, die gewählt sind aus der Gruppe bestehend aus Lignan(en), Flavonoid(en), Stilben(en) und deren Kombinationen.

15. Verfahren nach Anspruch 14, wobei diese Lignane gewählt sind aus Allohydroxymatairesinol, (-)-Hydroxymatairesinol, Secoisolariciresinol, Conidendrin, Conidendrinsäure, Todolactol, Isoliovil, Lariciresinol, Lignan A, Matairesinol, Oligolignanen, Nortrachelogenin, Pinoresinol und deren Kombinationen und die Stilbene gewählt sind aus Pinosylvin, Pinosylvin-Monomethylether und deren Kombinationen und wobei eine oder mehrere dieser Verbindungen aus dem Extrakt isoliert wird/werden.

16. Verfahren nach einem der vorhergehenden Ansprüche, wobei das lipophile Lösungsmittel nach der Extraktion rückgewonnen und zu einer anschließenden lipophilen Extraktion rückgeführt wird.

17. Verfahren nach einem der vorhergehenden Ansprüche, wobei die Extraktion mit einem lipophilen Lösungsmittel einen lipophilen Extrakt bereitstellt, der Bestandteile enthält, die gewählt sind aus der Gruppe bestehend aus Harzsäure(n), Fettsäure(n), Sterol(en), Juvabion(en), Triglycerid(en) und deren Kombinationen.

18. Verwendung einer lipophilen Flüssig-Flüssig-Extraktion zur Reinigung eines hydrophilen Knotenholzextrakts, wobei dieser hydrophile Knotenholzextrakt durch eine anfängliche Extraktion von Knotenholz mit einem hydrophilen Lösungsmittel gewonnen wird.

19. Verwendung nach Anspruch 18, wobei die Reinigung einen hydrophilen Knotenholzextrakt bereitstellt, der weniger als 25 %, bevorzugt weniger als 10 % und höchstbevorzugt weniger als 5 % Trockensubstanz an Verunreinigungen aufweist, die gewählt sind aus der Gruppe bestehend aus Harzsäuren, Fettsäuren, Sterolen, Juvabionen, Triglyceriden und deren Kombinationen.

20. Gereinigter Knotenholzextrakt, der sich durch Flüssig-Flüssig-Extraktion gewinnen lässt, **dadurch gekennzeichnet, dass** er mehr als 90 %, bevorzugt mehr als 95 % und höchstbevorzugt mehr als 99 % Trockensubstanz an Bestandteilen gewählt aus Kombinationen von Lignan(en), Flavonoid(en) und/oder Stilben(en) sowie weniger als 10 %, bevorzugt weniger als 5 % und höchstbevorzugt weniger als 1 % Trockensubstanz an Verunreinigungen gewählt aus Harzsäuren, Fettsäuren, Sterolen, Juvabionen, Triglyceriden und deren Kombinationen enthält.

21. Gereinigter Knotenholzextrakt nach Anspruch 20, im Wesentlichen bestehend aus 20-40 % Trockensubstanz an Stilbenen gewählt aus Pinosylvin und Pinosylvin-Monomethylether, aus 55-75 % Trockensubstanz an Lignanen gewählt aus Allohydroxymatairesinol, (-)-Hydroxymatairesinol, Secoisolariciresinol, Conidendrin, Conidendrinsäure, Todolactol, Isoliovil, Lariciresinol, Lignan A, Matairesinol, Oligolignanen, Nortrachelogenin und Pinoresinol und aus 1 bis 5 % Trockensubstanz an Verunreinigungen gewählt aus C18-Fettsäuren, Neoabietinsäure, Abietinsäure, Dehydroabietinsäure, Pimarsäure, Isopimarsäure und Palustrinsäure.

## Revendications

1. Procédé de fractionnement d'extrait de bois noueux, **caractérisé en ce que** le bois noueux est initialement extrait avec un solvant hydrophile, l'extrait de bois noueux résultant est extrait avec un solvant lipophile, au moins une fraction d'extrait de bois noueux est récupérée et le fractionnement est réalisé en tant qu'extraction liquide-liquide.

2. Procédé selon la revendication 1 dans lequel le solvant lipophile comprend un solvant non polaire ou un mélange de solvants non polaires ayant une constante diélectrique inférieure à 3 déterminée à 25 °C.

3. Procédé selon la revendication 2 dans lequel le solvant lipophile est choisi dans le groupe constitué par l'hexane, l'heptane, le benzène, le toluène, le cyclohexane, le cyclopentane et leurs mélanges.

4. Procédé selon la revendication 1 dans lequel le solvant hydrophile comprend un solvant polaire ou un mélange de solvants polaires ayant une constante diélectrique supérieure à 3, et préférablement supérieure à 15, déterminée à 25 °C.

5. Procédé selon la revendication 4 dans lequel le solvant hydrophile est choisi dans le groupe constitué par l'eau, l'alcool, l'acétone et leurs mélanges.

6. Procédé selon la revendication 1 dans lequel l'extraction avec un solvant hydrophile est réalisé sur un matériau de bois noueux non séché ou partiellement séché ayant une teneur en matière sèche de 60 à 95 %, préférablement de 70 à 90 %.

7. Procédé selon l'une des revendications précédentes dans lequel le solvant hydrophile utilisé pour l'extraction contient de l'eau.

8. Procédé selon la revendication 7 dans lequel la quantité d'eau dans le solvant hydrophile et/ou dans l'extrait de bois noueux hydrophile résultant est ajusté de manière à faciliter la séparation de phase de l'extraction à solvant lipophile, de préférence le solvant hydrophile comprenant de l'éthanol, le solvant lipophile comprenant de l'heptane et la teneur en eau étant de 10 % ou plus.

9. Procédé selon l'une des revendications précédentes dans lequel l'extraction avec un solvant lipophile est répétée au moins une fois.

10. Procédé selon l'une des revendications précédentes dans lequel la fraction d'extrait de bois noueux récupérée comprend une ou plusieurs fractions lipophiles et le solvant lipophile est éliminé par évaporation et la résine lipophile résultante est récupérée.

11. Procédé selon l'une des revendications précédentes dans lequel la fraction d'extrait de bois noueux récupérée comprend une ou plusieurs fractions hydrophiles et le solvant hydrophile est éliminé par évaporation et un extrait hydrophile purifié est récupéré et, en option, traité ultérieurement pour en dégager un ou plusieurs composants.

12. Procédé selon l'une des revendications précédentes dans lequel le bois noueux comprend des noeuds de bois de conifère de préférence choisi parmi le bois de pin (*Pinus*), d'épicéa (*Picea*), de sapin (*Abies*) et leurs combinaisons.

13. Procédé selon la revendication 12 dans lequel l'extraction avec un solvant hydrophile fournit un extrait hydrophile brut comprenant des composant(s) choisi(s) dans le groupe constitué par le(s) lignane(s), flavonoïde(s), stilbène(s), juvabione(s), acide(s) gras, acide(s) résinique(s), stérol(s), triglycéride(s) et leurs combinaisons.

14. Procédé selon la revendication 12 dans lequel l'extraction avec un solvant lipophile fournit un extrait hydrophile purifié comprenant plus de 90 % de la matière sèche de composant(s) choisi(s) dans le groupe constitué par le(s) lignane(s), flavonoïde(s), stilbène(s) et leurs combinaisons.

15. Procédé selon la revendication 14 dans lequel les lignanes sont choisis parmi l'allo-hydroxymatairésinol, le (-)-hydroxymatairésinol, le sécoisolaricirésinol, la conidendune, l'acide conidendrique, le todolactol, l'isoliovil, le laucirésinol, le lignane A, le matairésinol, les oligolignanes, la nortrachelogénine, le pinorésinol et leurs combinaisons, et les stilbènes sont choisis parmi la pinosylvine, le monométhyléther de pinosylvine et leurs combinaisons, et l'un ou plusieurs de ces composés est/sont isolé(s) de l'extrait.

16. Procédé selon l'une des revendications précédentes dans lequel le solvant lipophile est récupéré après l'extraction et recirculé vers une extraction lipophile ultérieure.

17. Procédé selon l'une des revendications précédentes dans lequel l'extraction avec un solvant lipophile fournit un extrait lipophile comprenant des composants choisis dans le groupe constitué par le(s) acide(s) résinique(s), acide(s) gras, stérol(s), juvabione(s), triglycéride(s) et leurs combinaisons.

18. Utilisation d'une extraction liquide-liquide lipophile pour la purification d'un extrait de bois noueux hydrophile, ledit extrait de bois noueux hydrophile étant obtenu par une extraction initiale de bois noueux avec un solvant hydrophile.

19. Utilisation selon la revendication 18 dans laquelle la purification fournit un extrait de bois noueux hydrophile ayant moins de 25 %, préférablement moins de 10 % et plus préférablement moins de 5 % de la matière sèche d'impuretés choisies dans le groupe constitué par les acides résiniques, acides gras, stérols, juvabiones, triglycérides et leurs combinaisons.

20. Extrait de bois noueux purifié obtenable par extraction liquide-liquide, **caractérisé en ce qu**'il contient plus de 90 %, préférablement plus de 95 % et plus préférablement plus de 99 % de la matière sèche de composants choisis parmi les combinaisons de lignane(s), de flavonoïde(s) et/ou de stilbène(s), et moins de 10 %, préférablement moins de 5 % et plus préférablement moins de 1 % de la matière sèche d'impuretés choisies parmi les acides résiniques, acides gras, stérols, juvabiones, triglycérides et leurs combinaisons.

21. Extrait de bois noueux purifié selon la revendication 20 composé essentiellement de 20 à 40 % de la matière sèche de stilbènes choisis parmi la pinosylvine et le monométhyléther de pinosylvine, de 55 à 75 % de la matière sèche de lignanes choisis parmi l'allo-hydroxymatairésinol, le (-)-hydroxymatairésinol, le sécoisolaricirésinol, la conidendrine, l'acide conidendrique, le todolactol, l'isoliovil, le laucirésinol, le lignane A, le matairésinol, les oligolignanes, la nortrachelogénine et le pinorésinol, et de 1 à 5 % de la matière sèche d'impuretés choisies parmi les acides gras C18, l'acide néoabiétique, l'acide abiétique, l'acide déhydroabiétique, l'acide pimarique, l'acide isopimarique et l'acide palustrique.
